## Europäisches Patentamt
## European Patent Office
## Office européen des brevets

(19)

(11) Veröffentlichungsnummer: **0 016 351**
**B1**

(12)

# EUROPÄISCHE PATENTSCHRIFT

(45) Veröffentlichungstag der Patentschrift:
04.05.83

(21) Anmeldenummer: **80100897.0**

(22) Anmeldetag: **25.02.80**

(51) Int. Cl.³: **G 08 B 21/00, G 01 N 27/12**

(54) **Gasmelder zum Einsatz in explosionsgefährdeter Umgebung.**

(30) Priorität: **16.03.79 CH 2482/79**

(43) Veröffentlichungstag der Anmeldung:
**01.10.80 Patentblatt 80/20**

(45) Bekanntmachung des Hinweises auf die Patenterteilung:
**04.05.83 Patentblatt 83/18**

(84) Benannte Vertragsstaaten:
**AT BE DE FR GB IT NL SE**

(56) Entgegenhaltungen:
**DE-A-1 498 580**
**DE-B-2 625 891**
**FR-A-1 367 009**
**FR-A-2 353 846**

(73) Patentinhaber: **CERBERUS AG, Alte Landstrasse 411,
CH-8708 Männedorf (CH)**

(72) Erfinder: **Christen, Peter, Im Wiesli 8,
CH-8708 Männedorf (CH)**
Erfinder: **Brändli, Hansheinrich, Rehbühlstrasse 29,
CH-8610 Uster (CH)**
Erfinder: **Durrer, Bernhard, Dorfstrasse 47,
CH-8712 Stäfa (CH)**
Erfinder: **Sauerbrey, Arnim, Frowiesstrasse 14,
CH-8344 Adetswil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Gasmelder zum Einsatz in explosionsgefährdeter Umgebung

Die vorliegende Erfindung betrifft einen Gasmelder zum Einsatz in explosionsgefährdeter Umgebung, der in Verbindung mit einer Gasmeldezentrale eine Gasmeldeanlage bildet und der einen bei Einwirkung reduzierender Gase seinen elektrischen Widerstand ändernden Gassensor enthält.

Zum Schutz von Betriebsstätten, Rohrleitungskanälen, Chemikalienlagern usw. vor Bränden sowie von Personen vor Vergiftungen, ist es wünschenswert, schädliche Konzentrationen von brennbaren bzw. giftigen Gasen möglichst frühzeitig zu erkennen, damit durch geeignete Gegenmaßnahmen, z. B. Abschalten schadhafter Betriebsanlagen, Stillegung undichter Rohrleitungen, Einschaltung von Ventilatoren, Verlassen gefährdeter Räume usw., Brände, Explosionen, Vergiftungen und andere Schädigungen vermieden werden können.

Zur Erkennung unerwünschter und gefährlicher Konzentrationen reduzierender, d. h. oxidierbarer oder brennbarer Gase gibt es Gasmeldeanlagen, die aus Gasmeldern bestehen, die an eine Gasmeldezentrale angeschlossen sind.

Bekannt sind weiter Gaswarngeräte, z. B. aus der DE-AS 2 625 891, die einen Diffusionsmeßkopf mit aktiven und inaktiven Gasspürelementen in einem mechanisch leicht lösbaren Gasspürelementeeinsatz unter einer Abdeckplatte aus gasdurchlässigem Sintermetall enthalten. Diese Gaswarngeräte verwenden zumeist eine Gasdetektor-Brückenschaltung, bei der zwei elektrisch beheizte — ein katalytisch aktives und ein inaktives — Thermoelemente gegeneinander geschaltet sind.

Es sind auch Gaswarngeräte bekannt, die als Gassensor Metalloxid-Halbleiter enthalten, die bei Einwirkung reduzierender Gase ihren elektrischen Widerstand ändern. Diese Widerstandsänderung wird in einer Auswerteschaltung in ein elektrisches Signal umgewandelt, durch das Alarmeinrichtungen betätigt und Gegenmaßnahmen eingeleitet werden.

Da die Arbeitstemperatur der Gassensoren meist in einem Temperaturbereich liegt, in dem die Entzündungstemperatur der nachzuweisenden Gase bereits weit überschritten ist (Äther und Acetaldehyd 180°C, Benzin und Dieselkraftstoff 220 – 300°C, Acetylen 305°C usw.), müssen Maßnahmen getroffen werden, die verhindern, daß die Gaswarngeräte die Entzündung der Gase bewirken, vor deren Anwesenheit sie warnen sollen. Zu diesem Zweck wurde vorgeschlagen, die Gassensoren mit einer Flammenbarriere aus einem engmaschigen Drahtgeflecht oder einem Gitter zu versehen. Diese Flammenbarrieren sind zwar als Schlagwetterschutz geeignet, stellen jedoch keinen wirksamen Explosionsschutz dar.

Es ist ferner vorgeschlagen worden, in dem zu überwachenden Bereich lediglich den Diffusionsmeßkopf in explosionsgeschützter Ausführung anzubringen und die Auswerteschaltung getrennt davon unterzubringen.

Der Einsatz von Halbleitern als Gassensoren in Gaswarngeräten ist verhältnismäßig neu. Die ersten brauchbaren Halbleiter-Gassensoren sind erst Anfang der siebziger Jahre bekanntgeworden (vgl. DE-OS 2 005 497 und 2 016 388). Ein wesentlicher Nachteil der verwendeten Gassensoren besteht darin, daß selbst innerhalb einer Produktionsserie große Empfindlichkeitsunterschiede bestehen, d. h. daß die Widerstandsänderung in Abhängigkeit von der Gaskonzentration bei den einzelnen Gassensoren nicht gleich ist. Damit eine sichere Alarmgebung erreicht werden kann, muß daher die Auswerteschaltung Schaltelemente enthalten, die es ermöglichen, die genannten Unterschiede auszugleichen.

Da andererseits die Lebensdauer der Gassensoren begrenzt ist, müssen sie in bestimmten Zeitabständen kontrolliert und gegebenenfalls ausgetauscht werden. Dies hat zur Folge, daß der Abgleich der Gassensoren am Einsatzort durchgeführt werden muß. Dazu muß der Gassensor in eine Atmosphäre mit einer bestimmten Konzentration an Prüfgas und Feuchtigkeit gebracht werden, während gleichzeitig die Auswerteschaltung abgeglichen wird. Da sich die Auswerteschaltung für die einzelnen Gasmelder in der Gaswarnzentrale befindet, muß der Abgleich der einzelnen Gassensoren dort vorgenommen werden, was offensichtlich erhebliche Nachteile aufweist. Diese Methode erfordert im allgemeinen einen zweiten Service-Monteur; bei großem Abstand zwischen Gasmelder und Zentrale gibt es zusätzlich Verständigungsschwierigkeiten.

Ein weiterer erheblicher Nachteil der bekannten Gaswarnanlagen besteht darin, daß die Sensoren am Einsatzort mit einem Eichgas an die Elektronik angepaßt werden müssen. Die im Handel erhältlichen Eichgase befinden sich in den Transportflaschen aus physikalischen Gründen nur im trockenen Zustand. Verwendet man jedoch zum Eichen trockene Gase, so sind die Sensoren erheblich weniger empfindlich. Bei einem korrekten Abgleich muß daher das Prüfgas befeuchtet werden, damit praxisnahe Bedingungen erzielt werden können. Ein wirklich korrekter Abgleich unter reproduzierbaren Bedingungen ist demgemäß nur im Herstellerwerk möglich. Eine Einstellung im Werk ist jedoch bei den bisher bekannten Gaswarnanlagen überhaupt nicht möglich, da die Länge der Leitungen zwischen Gassensor und Gaswarnzentrale nicht exakt bekannt ist, beim Abgleich der Gaswarngeräte jedoch berücksichtigt werden muß.

Es hätte nun nahegelegen, Auswerteschaltung und Gassensor zusammen in ein und demselben Gehäuse unterzubringen (Gasmelder) und zur Prüfung und zum Austausch der Gassensoren den ganzen Gasmelder in die Fabrik zu bringen. Dies bringt jedoch ebenfalls erhebliche Nachteile mit sich. Wegen der Bedingungen der Explosionssicherheit wäre der Gasmelder verhältnismäßig groß und unhandlich geworden; außerdem wäre die Explosionssicherheit nur schwer realisierbar und prüfbar gewesen. Die Auswerteschaltung wäre der Außenluft ausgesetzt gewesen, was in korrosiver Atmosphäre nicht tolerierbar gewesen wäre. Bei dieser Konstruktionsart hätte zudem der Gassensor nur bei offener Auswerteschaltung ausgewechselt werden können, so daß die Gefahr der Beschädigung und Verstellung bestanden hätte. Schon bei geringfügiger Beschädigung hätten die zulässigen Toleranzen für die Ex-Spalten nicht mehr gestimmt, so daß der Explosionsschutz nicht mehr gewährleistet wäre.

Ein weiterer Nachteil wäre, daß sich explosionsgeschützt, d. h. druckfest gekapselte Räume nur schwer öffnen lassen und daß diese Schwierigkeiten zunehmen, je größer diese Räume sind. Ein wesentlicher Nachteil besteht noch darin, daß der Austausch der Gasmelder am Einsatzort ziemlich mühsam ist, da auch die elektrischen Anschlüsse der Gasmelder an das Leitungsnetz in explosionsgeschützter Ausführung erstellt werden müssen, d. h. schwierig zu lösen sind.

Aufgabe der Erfindung ist die Beseitigung der erwähnten Nachteile und die Schaffung eines Gasmelders zum Einsatz in explosionsgefährdeter Umgebung, der in Verbindung mit einer Gasmeldezentrale eine Gasmeldeanlage bildet, und der einen bei Einwirkung reduzierender Gase seinen elektrischen Widerstand ändernden Gassensor enthält, und bei dem Gassensor und Auswerteschaltung in einem einzigen, explosionsgeschützten Gehäuse als Gasmelder zusammengefaßt sind. Weitere Aufgaben, welche die Erfindung löst, bestehen darin, den Gasmelder so .auszubilden, daß es möglich ist, den Gassensor auszuwechseln, ohne die Auswerteschaltung der Außenatmosphäre zugänglich zu machen und bei dem es außerdem möglich ist, den Abgleich der Gassensoren in der Fabrik des Herstellers durchzuführen.

Erfindungsgemäß wird dies dadurch erreicht, daß der Gasmelder in einem Gehäuse mindestens zwei voneinander getrennte Räume aufweist, von denen der eine, druckfest gekapselte Raum die elektronische Auswerteschaltung enthält und der zweite, in explosionsgeschützter Bauart ausgeführte Raum, der durch eine Abdeckung aus gasdurchlässigem Sintermetall, durch die Gasaustausch mit der Atmosphäre möglich ist, abgeschlossen ist, den Gassensor mit einem Abgleichwiderstand enthält.

Die Begriffe »druckfest gekapselt« und »explosionsgeschützt« haben in der ganzen Beschreibung sowie in den Patentansprüchen die übliche Bedeutung im Sinne von Vorschriften der staatlichen Verbände, z. B. der »Vorschriften für explosionsgeschützte elektrische Betriebsmittel« des Verbandes deutscher Elektrotechniker (VDE), VDE-Vorschrift 0171/2.61.

Gemäß einer bevorzugten Ausführungsform bildet der Gassensor zusammen mit dem Abgleichwiderstand eine Einheit, wobei der Abgleichwiderstand so ausgebildet ist, daß alle Gassensor/Abgleichwiderstand-Einheiten — trotz der unterschiedlichen Eigenschaften der einzelnen Gassensoren — stets die gleichen elektrischen Eigenschaften aufweisen (gleiches Verhältnis von Widerstand $R_G$ des Gassensors zu Widerstand $R_A$ des Abgleichwiderstandes, d. h. $R_G : R_A$ = const.).

Die Gassensor/Abgleichwiderstand-Einheit kann beispielsweise so ausgebildet sein, daß Gassensor und Abgleichwiderstand nicht (oder außerordentlich schwer) voneinander getrennt werden können. Die Gassensor/Abgleichwiderstand-Einheit weist dabei an der Seite, welche bei stehender Anordnung die Unterseite (bei hängender die Oberseite) ist, Stecker auf, die gleichzeitig zur mechanischen Verbindung mit dem Gehäuse und zur elektrischen Verbindung mit der Auswerteschaltung dienen. Vorzugsweise befindet sich die Steckvorrichtung für die Gassensor/Abgleichwiderstand-Einheit in dem Deckel, der den Raum mit der Auswerteschaltung abschließt.

Diese Art der Ausführung ermöglicht es, auf den Abgleich des Gassensors am Einsatzort zu verzichten. Der Abgleichwiderstand wird in der Fabrik des Herstellers auf den Gassensor abgestimmt und mit diesem zu einer Einheit verbunden. Zur Kontrolle der Empfindlichkeit werden die Gassensor/Abgleichwiderstand-Einheiten ausgewechselt und in die Fabrik verbracht, wo die Überprüfung unter kontrollierten Bedingungen leicht möglich ist. Der Gasmelder kann am Einsatzort verbleiben und die Auswerteschaltung kommt beim Auswechseln nicht mit der Atmosphäre in Berührung.

Gemäß einer weiteren bevorzugten Ausführungsform des erfindungsgemäßen Gasmelders wird als Gassensor ein üblicher Halbleiter-Gassensor verwendet, der in Gegenwart von oxidierbaren Gasen seinen elektrischen Widerstand ändert. Mit diesen Gassensoren können z. B. brennbare Gase im Konzentrationsbereich von einigen Prozent der unteren Explosionsgrenze (UEG) zuverlässig erkannt werden. Mit diesen Gassensoren können toxische und/oder brennbare Gase im Gemisch mit Luft erkannt werden, beispielsweise Methan, Propan, Butan, Erdgas, Spaltgas, Wasserstoff oder Kohlenmonoxid, aber auch Dämpfe organischer Flüssigkeiten, z. B. von Alkoholen, Ketonen, Estern, Äthern; Beispiele für Dämpfe toxischer Verbindungen sind u. a. Toluol-2,4-diisocyanat (TDI), Vinylchlorid und Schwefelwasserstoff. Auch andere oxidierbare Gase, wie z. B. Ammoniak können mit diesen Gassensoren erkannt werden.

Ein solcher Gassensor kann beispielsweise ein Halbleiter sein, der in einem Temperaturbereich von $200-450°C$ bei Einwirkung reduzierender Gase seinen Widerstand ändert und der mit einem Abgleichwiderstand gekoppelt ist, dessen Widerstand so gewählt ist, daß bei der Arbeitstemperatur des Gassensors das Verhältnis des Widerstandes des Gassensors zum Widerstand des Abgleichwiderstandes einen vorbestimmten Wert aufweist. Der Abgleichwiderstand kann beispielsweise im Bereich von 50 – 270 Ohm liegen.

Anstelle der vorstehend erwähnten Halbleiter-Gassensoren können auch »Pellistoren« verwendet werden, d. h. katalytische Elemente, auch »katalytische Transducer« genannt, die eine kurze Spule aus Platindraht enthalten, die in ein Kügelchen (»pellet«) aus einem refraktären Oxid, z. B. Aluminiumoxid, eingebettet ist. Kommt ein oxidierbares Gas mit dem Kügelchen in Berührung, so verbrennt es an dessen Oberfläche und erhöht die Temperatur des Platindrahtes weiter, wodurch eine Änderung des elektrischen Widerstands erfolgt. Diese Widerstandsänderung wird in einer abgestimmten Brückenschaltung (Wheatstone'sche Brücke) ausgewertet und kann zur Konzentrationsbestimmung oxidierbarer Gase dienen. Werden anstelle der vorstehend genannten Halbleiter-Gassensoren solche Pellistoren verwendet, kann die Auswerteschaltung leicht den geänderten elektrischen Eigenschaften der Pellistoren angepaßt werden.

Gemäß einer weiteren, bevorzugten Ausführungsform des erfindungsgemäßen Gasmelders ist die Abdeckung des zweiten Raums, in dem sich die Gassensor/Abgleichwiderstand-Einheit befindet in Form einer zylindrischen, unten offenen Büchse ausgebildet, deren Wand und Deckel aus gasdurchlässigem Sintermetall besteht. Das Material ist vorzugsweise Chrom-Nikkel-Molybdän-Sintermetall, insbesondere mit einer Filterfeinheit von etwa 30 μm.

Um eine einfache Anpassung des erfindungsgemäßen Gasmelders an unterschiedliche Gase und/oder Konzentrationsbereiche zu ermöglichen, weisen die Gasmelder vorzugsweise einen ohne Öffnen des Gerätes mit Spezialwerkzeug zugänglichen, unter Spannung umschaltbaren Schalter zur Empfindlichkeitseinstellung auf.

Die Verbindung der erfindungsgemäßen Gasmelder mit der Gasmeldezentrale erfolgt über Leitungen, wobei der Anschluß der Leitungen an den Gasmelder vorzugsweise in einem dritten, explosionsgesicherten, verschraubbaren, mit den beiden anderen Räumen nicht in Verbindung stehenden Raum innerhalb des Gehäuses der Gasmelder erfolgt.

Zur Anzeige, welcher Gasmelder angesprochen hat, weist eine weitere bevorzugte Ausführungsform des erfindungsgemäßen Gasmelders einen explosionsgeschützten Ansprechindikator, vorzugsweise direkt auf dem Raum zur Aufnahme der Anschlußklemmen, auf.

Um die aus Sintermetall bestehende Abdeckung des den Gassensor enthaltenden Raumes vor Spritzwasser und Verstaubung zu schützen, kann man den Gasmelder mit einer Spritzschutzhaube, vorzugsweise einer Metallhaube mit vergitterten Fenstern, versehen.

Ein wesentlicher Vorteil des erfindungsgemäßen Gasmelders besteht darin, daß er Auswerteschaltung und Gassensor innerhalb eines Gasmelders enthält. Dadurch ist es möglich, eine Vielzahl von Gasmeldern gruppenweise zusammengefaßt (vorzugsweise bis zu 10 Gasmelder pro Gruppe) an die Gasmeldezentrale anzuschließen. Dadurch wird eine erheblich einfachere Leitungsführung ermöglicht, da die bei den bisher bekannten Gasmeldeanlagen erforderlichen individuellen Anschlüsse jedes einzelnen Gasmelders mit einer Vielzahl von Leitungen an die Gasmeldezentrale entfällt. Bei den bisher bekannten Gaswarngeräten sind pro Gasmelder für jeden einzelnen Gassensor mindestens drei Leitungen erforderlich, die bis zur Zentrale geführt werden müssen, während durch Verwendung des erfindungsgemäßen Gasmelders nur insgesamt fünf Leitungen pro Gasmeldergruppe erforderlich sind. Dies stellt eine erhebliche Vereinfachung dar.

Die Erfindung wird anhand von Ausführungsbeispielen näher erläutert. Es zeigt

Fig. 1 einen erfindungsgemäßen Gasmelder in hängender Anordnung im Querschnitt,

Fig. 2 eine erfindungsgemäße Gassensor/Abgleichwiderstand-Einheit (in stehender Anordnung) in getrenntem Zustand, wobei Fig. 2a den Gassensor in perspektivischer Darstellung von unten-seitlich und Fig. 2b den Abgleichwiderstand in gleicher Darstellung (zur besseren Erkennbarkeit halbdurchsichtig) zeigen,

Fig. 3a bis 3d Schaltungsanordnungen für den Schalter zur Empfindlichkeitseinstellung des erfindungsgemäßen Gasmelders und

Fig. 4 die Schaltungsanordnung der Gassensor/Abgleichwiderstand-Einheit.

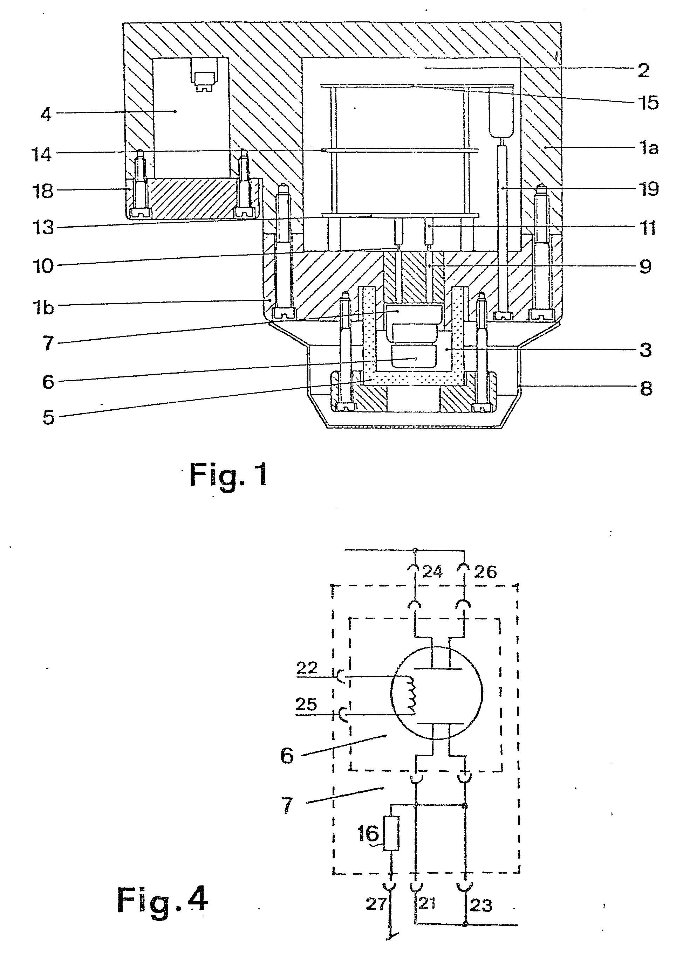

Fig. 1 zeigt den prinzipiellen Aufbau einer Ausführungsform eines erfindungsgemäßen Gasmelders, und zwar mit drei voneinander getrennten, explosionsgeschützten bzw. explosionsgesicherten Räumen 2, 3 und 4. In einem Körper 1a und Deckel 1b aufweisenden Gehäuse 1, z. B. aus Stahl, Aluminium oder Kunststoff, befindet sich ein erster druckfest gekapselter Raum 2, in dem sich die Auswerteschaltung befindet (»Schaltungsraum«).

Der Deckel 1b schließt den Raum 2 nach oben (bei hängender Anordnung: nach unten) ab und bildet den Boden des zweiten in explosionsgeschützter Bauart ausgeführten Raums 3, der den Gassensor 6 und den Abgleichwiderstand 7 enthält (»Sensorraum«) und durch die Abdeckung 5 aus gasdurchlässigem Sintermetall mit dem zu überwachenden Raum in Verbindung steht. Im Körper 1a des Gehäuses befindet sich außerdem der dritte, explosionsgesicherte Raum 4, der die Anschlußklemmen aufnimmt (»Anschlußraum«) und durch den Anschlußraumdeckel 18 hermetisch von der Außenluft abgeschlossen wird.

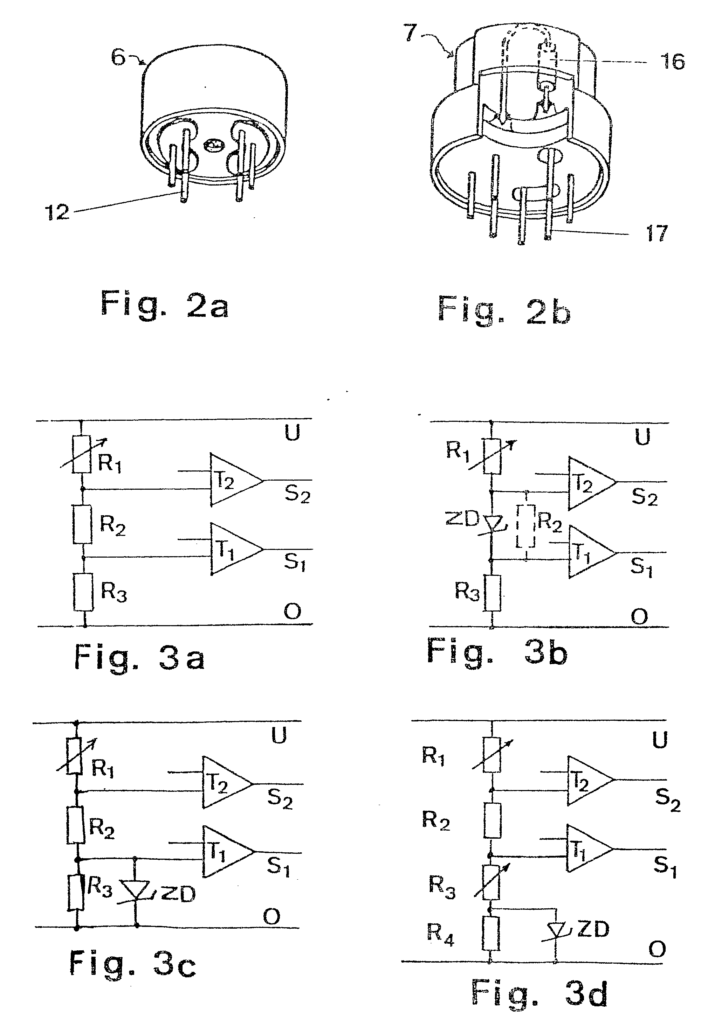

Der Sensorraum 3 enthält nur den Gassensor 6 und den Abgleichwiderstand 7, deren Aufbau zusätzlich in den Fig. 2a und 2b näher erläutert ist. Der Gassensor 6 weist an der Unterseite Kontaktstifte 12 auf, die beim Zusammenstecken in entsprechende (nicht dargestellte) Buchsen an der Oberseite des Abgleichwiderstands 7 eingreifen und die mechanische und elektrische Verbindung zwischen den beiden Teilen herstellen.

Gemäß einer bevorzugten Ausführungsform wird die Verbindung durch Federkontakte so ausgestaltet, daß eine Trennung der Teile außerordentlich erschwert, wenn nicht unmöglich gemacht wird. Dadurch wird erreicht, daß eine unbefugte Trennung des Abgleichwiderstandes 7 vom zugehörigen Gassensor 6, für dessen Abgleich er ausgelegt ist, verhindert wird.

Der Abgleichwiderstand 7 weist an der Unterseite Kontaktstifte 17 auf, mit denen die Gassensor/Abgleichwiderstand-Einheit in Metallbuchsen 9, die sich im Gehäusedeckel 1b befinden, eingesteckt wird und durch welche die mechanische Verbindung mit dem Gehäuse 1 und gleichzeitig die elektrische Verbindung mit der Auswerteschaltung hergestellt wird. Dazu weisen die mittels eines Gießharzes in den Deckel 1b eingelassenen Buchsen 9 an der im Raum 2 befindlichen Unterseite des Deckels 1b Kontaktstifte 10 auf, an die mittels eines Buchsensteckers 11 die Auswerteschaltung des Gasmelders angeschlossen ist.

In dem in Fig. 2b dargestellten Abgleichwiderstand-Teil 7 der Einheit 6/7 befindet sich der eigentliche Abgleichwiderstand 16, durch den erreicht wird, daß sämtliche Gassensor/Abgleichwiderstand-Einheiten 6, 7 die gleichen elektrischen Eigenschaften haben. Dazu ist der Abgleichwiderstand 16 so geschaltet, daß er beim Zusammenstecken mit dem Gassensor 6 einen Spannungsteiler bildet, an dessen Abgriff das Signal abgenommen wird, das der Auswerteschaltung zugeführt wird.

Die Schaltung der Gassensor/Abgleichwiderstand-Einheit ist an einem Beispiel in Fig. 4 dargestellt. Zwischen den Anschlüssen 24 und 26 (aus Sicherheitsgründen doppelte Stecker) und dem Anschluß 27 liegen der Gassensor 6 und der Abgleichwiderstand 16. Gassensor 6 und Abgleichwiderstand 16 bilden einen Spannungsteiler, an dessen Abgriff das Signal abgenommen wird, welches über die ebenfalls im Doppel ausgeführten Stecker 21 und 23 der Auswerteschaltung zugeführt wird, wobei der Widerstand 16 so gewählt wird, daß das Verhältnis des Abgleichwiderstandes 16 zu dem Widerstand des Gassensors 6 für alle Gassensor/Abgleichwiderstand-Einheiten gleich ist. Bei gleicher Speisespannung ist in diesem Fall die Spannung an den Anschlüssen 21, 23 bei gleichen Umgebungsbedingungen für alle Gassensor/Abgleichwiderstand-Einheiten gleich. Die Stromzufuhr für die Heizung des Gassensors 6 erfolgt über die Anschlüsse 22 und 25.

In dem druckfest gekapselten Raum 2 (Fig. 1) befindet sich die Auswerteschaltung. Diese ist zweckmäßigerweise auf mehrere, übereinander angeordnete Platten verteilt. Die Schaltungselemente sind dabei so auf die einzelnen Platten verteilt, daß bei einer gegebenenfalls erforderlich werdenden Schaltungsänderung möglichst nur eine Platte ausgewechselt werden muß. Dies kann beispielsweise der Fall sein, wenn man den Halbleiter-Gassensor durch einen Gassensor austauscht, der nach dem Prinzip der katalytischen Oxidation arbeitet, wodurch eine abgestimmte Meßbrückenschaltung in der Auswerteschaltung erforderlich ist.

Auf einer obersten (bei hängender Anordnung: untersten) Platte — der sogenannten »Inverterplatte« 13 — sind Schaltungselemente angeordnet, die das von dem Gassensor abgegebene Signal verstärken und für die Weiterleitung geeignet machen. Ferner enthält diese Platte Schaltelemente zur Einstellung, Stabilisierung und Überwachung der Sensorbetriebsspannung und des zum Heizen des Gassensors erforderlichen Stroms sowie zur eventuellen Erzeugung eines Störungsanzeigesignals. Bei Unterbrechung des Stroms, bei Kurzschluß oder bei einem wesentlichen Mehr- oder Wenigerverbrauch erfolgt Abgabe eines Störungsanzeigesignals auf der entsprechenden Signalleitung.

Die mittlere Platte — die sogenannte »Logikplatte« 14 — enthält Schaltungselemente, welche die Auswertung des Sensorsignals ermöglichen, z. B. die Schwellenwertschalter für Vorwarn- und Alarmsignal oder die Weiterleitung eines Störsignals. Außerdem sind auch Schaltkreise vorgesehen, die das Ansprechen von mehr als einem Gasmelder derselben Gruppe verhindern zusätzlich Ansteuerung für Ansprechindikator.

Die unterste (bei hängender Anordnung: oberste) Platte — die sogenannte »Grundplatte« 15 — enthält Schutzvorrichtungen für den Gasmelder, z. B. Sicherungen gegen Fehlanschlüsse, falsche Polarität, Überspannungen etc. sowie gegebenenfalls einen Schalter zur Veränderung der Empfindlichkeit des Gasmelders.

Gasmeldeanlagen sind im allgemeinen zweistufig ausgebildet, d. h. es erfolgt bei einer bestimmten Gaskonzentration eine »Vorwarnung«, während bei einer höheren Gaskonzentration »Alarm« ausgelöst wird. Es ist wünschenswert, daß die Schwellenwerte für »Vorwarnung« und »Alarm« eines Gasmelders bestimmten Anwendungszwecken angepaßt werden können. Dazu dienen Einstelleinrichtungen, wie sie in den Fig. 3a bis 3d dargestellt sind; mit diesen Einstelleinrichtungen können beide Schwellenwerte eines Gasmelders gleichzeitig eingestellt werden. Sie bestehen aus jeweils einem Spannungsteiler zwischen der Speiseleitung U und dem Nulleiter, welcher bei dem Beispiel nach Fig. 3a aus drei Widerständen $R_1$, $R_2$ und $R_3$ besteht, wovon der erste Widerstand $R_1$ einstellbar ist. Bei dem Beispiel nach Fig. 3b ist der Widerstand $R_2$ durch eine Zenerdiode ZD ersetzt oder zu einer Zenerdiode ZD parallel geschaltet. In Fig. 3c ist zum Widerstand $R_3$ eine Zenerdiode ZD parallel geschaltet. Die beiden Abgriffe am Spannungsteiler sind mit den Referenzeingängen von zwei Schwellenwertschaltern $T_1$ und $T_2$ verbunden, deren Steuereingänge das Analog-Ausgangssignal des zugehörigen Gassensors entsprechend der Widerstandsänderung bei Gaseinwirkung erhalten und deren Ausgänge mit den Signalleitungen $S_1$ und $S_2$ verbunden sind. Durch Verstellung des Widerstands $R_1$ erreicht man im Beispiel nach Fig. 3a eine gleichzeitige proportionale Verschiebung beider Konzentrationsschwellen, während bei dem Beispiel nach Fig. 3b eine Parallelverschiebung beider Schwellenwerte zumindest in einem Teil des Einstellbereiches stattfindet. Bei dieser Kombination ist es möglich, Nichtlinearitäten der Gasfühlerelemente weitgehend auszugleichen. Im Beispiel nach Fig. 3c verschieben sich zunächst beide Schwellenwerte proportional, bis die Durchbruchspannung der Zenerdiode erreicht ist. Von diesem Wert an bleibt der untere Schwellenwert konstant, und zwar auf dem Wert, der durch die Zenerdiode ZD festgelegt ist. Verbindet man, wie in Fig. 3d dargestellt, die Zenerdiode ZD mit dem Abgriff eines aus zwei Widerständen $R_3$ und $R_4$ gebildeten Spannungsteilers, d. h. spaltet man den Widerstand $R_3$ der Fig. 3c in zwei Komponenten auf und verbindet die Zenerdiode mit dem Abgriff, wobei mindestens eine dieser Komponenten einstellbar ist, so kann eine weitere, empfindlichere Einstellmöglichkeit des Schwellenwertes erreicht werden.

Eine wichtige Anforderung an Gasmeldeanlagen ist, daß diese in ihrer Empfindlichkeit verschiedenen Einsatzbedingungen angepaßt werden können und daß die eingestellte Empfindlichkeit nicht zufällig oder durch nichtautorisiertes Personal verstellt werden kann. Dies ist bei dem erfindungsgemäßen Gasmelder dadurch gewährleistet, daß die Einstellung der einzelnen Gasmelder nicht wie bei den bisher bekannten Gaswarngeräten durch Einstellung eines Potentiometers am Einsatzort erfolgen muß, sondern daß diese Einstellung mittels des obenerwähnten Schalters auf vom Werk fest vorgegebene Empfindlichkeitsstufen erfolgt.

Gemäß einer bevorzugten Ausführungsform ist ein mit einem Spezialschlüssel zu betätigender Schalter am Gerät vorgesehen, mit dessen Hilfe vier verschiedene Empfindlichkeitsstufen des Gasmelders eingestellt werden können. Vier Empfindlichkeitsstufen sind für die Praxis ausreichend.

Gemäß einer weiteren bevorzugten Ausführungsform des erfindungsgemäßen Gasmelders ist am Meldergehäuse bzw. in unmittelbarer Nähe des Gasmelders ein Ansprechindikator angebracht, der den jeweiligen Zustand des Gasmelders anzeigt. Üblicherweise wird die Vorwarnstufe durch dauerndes Aufleuchten einer Lampe, der Alarmzustand durch ständiges Aufblinken dieser Lampe angezeigt, und durch den gleichen Ansprechindikator können auch Störungen der Ansprechbereitschaft des Gasmelders angezeigt werden. Dies hat den Vorteil, daß das im Raum anwesende Personal jederzeit über bedenkliche bzw. gefährliche Gaskonzentrationen durch den Gasmelder informiert wird.

Die wesentlichen Vorteile des erfindungsgemäßen Gasmelders bestehen darin, daß durch die Aufnahme der Auswerteschaltung in einen vom Sensor getrennten Raum innerhalb des Gasmelders ein erheblich einfacher Aufbau der gesamten Gasmeldeanlage, eine erheblich einfachere Projektierung, Wartung und Überprüfung der Gasmelder sowie eine erheblich einfachere Einstellung und Überprüfung der Ansprechbereitschaft der Gasmelder erfolgen kann.

## Patentansprüche

1. Gasmelder zum Einsatz in explosionsgefährdeter Umgebung, der in Verbindung mit einer Gasmeldezentrale eine Gasmeldeanlage bildet und der einen bei Einwirkung reduzierender Gase seinen elektrischen Widerstand ändernden Gassensor (6) enthält, dadurch gekennzeichnet, daß der Gasmelder in einem Gehäuse (1) mindestens zwei voneinander getrennte Räume (2, 3) aufweist, von denen der eine, druckfest gekapselte Raum (2) die elektronische Auswerteschaltung enthält und der zweite, in explosionsgeschützter Bauart ausgeführte Raum (3), der durch eine Abdeckung (5) aus gasdurchlässigem Sintermetall, durch die Gasaustausch mit der Atmosphäre möglich ist, abgeschlossen ist, den Gassensor (6) mit einem Abgleichwiderstand (7; 16) enthält.

2. Gasmelder gemäß Patentanspruch 1, dadurch gekennzeichnet, daß der Gassensor (6) mit dem Abgleichwiderstand (7; 16) zusammen eine Einheit (6, 7/16) bildet, und daß diese Sensor/Abgleichwiderstand-Einheit (6, 7/16) steckbar im Deckel (1b) des Gehäuses (1), der den einen Raum (2) abschließt, angeordnet ist.

3. Gasmelder gemäß Patentanspruch 2, dadurch gekennzeichnet, daß der Gassensor (6) ein Halbleiter ist, der in einem Temperaturbereich von $200-450°C$ bei Einwirkung reduzierender Gase seinen Widerstand ändert und daß der Abgleichwiderstand (16) so gewählt ist, daß das Verhältnis des Widerstands des Gassensors (6) zum Widerstand des Abgleichwiderstands (16) bei der Arbeitstemperatur des Halbleiters und einer vorgewählten Gaskonzentration einen vorbestimmten Wert hat.

4. Gasmelder gemäß Patentanspruch 3, dadurch gekennzeichnet, daß der Abgleichwiderstand (16) einen elektrischen Widerstand von $50-270$ Ohm aufweist.

5. Gasmelder gemäß Patentanspruch 1, dadurch gekennzeichnet, daß er einen nach dem Prinzip der katalytischen Oxidation arbeitenden Gassensor enthält und daß er zur Auswertung der Widerstandsänderung eine abgeglichene Brückenschaltung aufweist, wobei der Abgleich und die Brückenverstärkung analog zum Gassensor in einer steckbaren Adapter-Einheit mit Abgleichwiderständen vorgenommen wird.

6. Gasmelder gemäß Patentanspruch 1, dadurch gekennzeichnet, daß die Abdeckung (5) eine Büchse aus Sintermetall, die den Gassensor umgibt, vorzugsweise aus rostfreiem Cr−Ni−Mo-Sintermetall ist, und daß die Filterfeinheit des Sintermetalls vorzugsweise etwa 30 μm beträgt.

7. Gasmelder gemäß Patentanspruch 1, dadurch gekennzeichnet, daß er zur Empfindlichkeitseinstellung einen ohne Öffnung des Gehäuses zugänglichen, unter Spannung umschaltbaren, mit Spezialwerkzeug bedienbaren Schalter (19) aufweist.

8. Gasmelder gemäß Patentanspruch 1, dadurch gekennzeichnet, daß er einen dritten, explosionsgesicherten, verschraubbaren Raum (4) zur Aufnahme der Anschlußklemmen aufweist.

9. Gasmelder gemäß Patentanspruch 1 oder 8, dadurch gekennzeichnet, daß er einen explosionsgesicherten Ansprechindikator, vorzugsweise direkt auf dem Raum (4) zur Aufnahme der Anschlußklemmen montiert, aufweist.

10. Gasmelder gemäß Patentanspruch 1, dadurch gekennzeichnet, daß er eine Spritzschutzhaube (8), vorzugsweise eine Metallhaube mit vergitterten Fenstern, zum Schutz vor Verstaubung und Verstopfung der Poren des Sintermetalls aufweist.

## Claims

1. A gas sensing unit for use in explosion endangered invironments, which in conjunction with a gas sensing central station forms a gas sensing signalling installation and which contains a gas sensor (6) which alters its electrical resistance under the action of reducing gases, characterized by the features that, the gas sensing unit contains within a housing (1) at least two separated chambers (2, 3), wherein the one compression-proof encapsulated chamber (2) contains the electronic evaluation circuit and the second explosion-protected designed chamber (3), which is a covered by a cover (5) formed of a gas-pervious sintered metal through which there is possible the gas exchange with the atmosphere, contains the gas sensor (6) with a balancing resistance (7; 16).

2. The gas sensing unit according to patent claim 1, characterized by the features that the gas sensor (6) together with the balancing resistance (7; 16) conjointly forms a unit (6, 7, 16), and that this sensor/balancing resistance unit (6, 7, 16) is arranged to be pluggable into the cover (1b) of the housing (1) which closes the one chamber (2).

3. The gas sensing unit according to patent claim 2, characterized by the features that the gas sensor (6) comprises a semiconductor which alters its resistance in a temperature range of 200° −450°C in the presence of reducing gases and that the balancing resistance (16) is selected such that the ratio of the resistance of the gas sensor (6) to the resistance of the balancing resistance (16) at the working temperature of the semiconductor and a preselected gas concentration has a predetermined value.

4. The gas sensing unit according to patent claim 3, characterized by the features that the balancing resistance (16) possesses an electrical resistance of 50−270 ohms.

5. The gas sensing unit according to patent claim 1, characterized by the features that it contains a gas sensor working according to the principle of catalytic oxidation and that it possesses a balanced bridge circuit for evaluation of the resistance change, wherein the balancing and the bridge amplification are performed analogous to the gas sensor in a plug-connectable adapter unit containing balancing resistances.

6. The gas sensing unit according to patent claim 1, characterized by the features that the cover (5) comprises a bushing formed of sintered metal, which surrounds the gas sensor, preferably is formed of stainless Cr−Ni−Mo-sintered metal and that the filter fineness of the sintered metal preferably amounts to approximately 30 μm.

7. The gas sensing unit according to patent claim 1, characterized by the features that it contains a switch (19) for sensitivity adjustment and which is accessible without opening the housing and can be switched by applying a potential thereto and can be serviced with a special tool.

8. The gas sensing unit according to patent claim 1, characterized by the features that it contains a third, explosion protected threadably connectable chamber (4) for the reception of the connection terminals.

9. The gas sensing unit according to patent claim 1 or 8, characterized by the features that it possesses àn explosion protected response indicator, preferably mounted directly at the chamber (4) for the reception of the connection terminals.

10. The gas sensing unit according to patent claim 1, characterized by the features that it possess a splash-proof protective hood (8), preferably a metal hood having gridded windows for the protection against dust contamination and clogging of the pores of the sintered metal.

## Revendications

1. Appareil détecteur de gaz destiné à être utilisé dans un environnement explosif, qui constitue une installation de détection de gaz en liaison avec une centrale de détection de gaz, et qui comporte un détecteur de gaz (6) dont la résistance électrique varie sous l'action de gaz réducteurs, caractérisé par le fait que l'appareil détecteur de gaz comporte, dans un boîtier (1), au moins deux chambres (2, 3) isolées l'une de l'autre, dont la première chambre (2), munie d'un blindage à l'épreuve de la pression, contient le circuit d'évaluation électronique, et la seconde chambre (3), possédant une constitution antidéflagrante, qui est fermée par un couvercle (5) un métal fritté perméable aux gaz, à travers lequel un échange de gaz avec l'atmosphère est possible, contient le détecteur de gaz (6) avec une résistance de réglage (7; 16).

2. Appareil détecteur de gaz suivant la revendication 1, caractérisé par le fait que le détecteur de gaz (6) constitue une unité (6, 7; 16) avec la résistance de réglage (7; 16), et que cette unité détecteur de gaz/résistance de réglage (6, 7; 16) est enfichée dans le couvercle (1b) du boîtier (1), qui ferme une des chambres (2).

3. Appareil détecteur de gaz suivant la revendication 2, caractérisé par le fait que le détecteur de gaz (6) est un semiconducteur dont la résistance varie sous l'effet de gaz réducteurs dans une gamme de températures allant de 200 à 450°C, et que la résistance de réglage (16) est choisie de manière que le rapport de la résistance du détecteur de gaz (6) à la résistance de la résistance de réglage (16) ait une valeur prédéterminée à la température de travail du semiconducteur et à une concentration de gaz présélectionnée.

4. Appareil détecteur de gaz suivant la revendication 3, caractérisé par le fait que le résistance de réglage (16) possède une résistance électrique de 50 à 270 ohms.

5. Appareil détecteur de gaz suivant la revendication 1, caractérisé par le fait qu'il contient un détecteur de gaz fonctionnant suivant le principe de l'excitation catalytique, et qu'il comporte un circuit en pont équilibré pour l'évaluation de la variation de la résistance, le réglage et l'amplification du pont étant effectués de façon analogue au détecteur de gaz, dans une unité d'adaptation enfichable comportant des résistances de réglage.

6. Appareil détecteur de gaz suivant la revendication 1, caractérisé par le fait que le couvercle (5) est une douille en métal fritté, qui entoure le détecteur de gaz, de préférence en métal fritté inoxydable à base de Cr — Ni — Mo, et que la finesse de filtrage du métal fritté est égale de préférence à environ 30 µm.

7. Appareil détecteur de gaz suivant la revendication 1, caractérisé par le fait que pour le réglage de la sensibilité il comporte un commutateur (19) qui peut être actionné à l'aide d'un outil spécial, qui peut être mis sous tension, et qui est accessible sans ouverture du boîtier.

8. Appareil détecteur de gaz suivant la revendication 1, caractérisé par le fait qu'il comporte une chambre (4) antidéflagrante, qui peut être vissée, pour recevoir les bornes de raccordement.

9. Appareil détecteur de gaz suivant la revendication 1 ou 8, caractérisé par le fait qu'il comporte un indicateur de réponse antidéflagrant, monté de préférence directement sur la chambre (4) destinée à recevoir les bornes de raccordement.

10. Appareil détecteur de gaz suivant la revendication 1, caractérisé par le fait qu'il comporte un capuchon de protection vis-à-vis des projections (8), de préférence un capot métallique avec une fenêtre grillagée, pour protéger les pores du métal fritté vis-à-vis d'un encrassement ou d'un engorgement.

Fig. 1

Fig. 4

Fig. 2a

Fig. 2b

Fig. 3a

Fig. 3b

Fig. 3c

Fig. 3d